# EUROPEAN PATENT APPLICATION

(11) **EP 2 492 318 A1**
(43) Date of publication of application: **29.08.2012**
(21) Application number: 10824876.6
(22) Date of filing: 15.10.2010
(51) Int. Cl.: C09B 57/10, C07D 213/22, C07D 213/79, C07D 235/20, H01L 31/04, H01M 14/00, C07F 15/00

(54) **PHOTOELECTRIC CONVERSION DEVICE WHEREIN DYE CONSISTING OF BINUCLEAR RUTHENIUM COMPLEX HAVING SUBSTITUTED BIPYRIDYL GROUPS IS USED, AND PHOTOCHEMICAL CELL**

(30) Priority: 20.10.2009 JP 2009241793
(71) Applicant: Ube Industries, Ltd., Ube-shi Yamaguchi 755-8633 (JP)
(72) Inventor: KAKITA, Kazuaki, Ichihara-shi Chiba 290-0045 (JP); IWASA, Takafumi, Ichihara-shi Chiba 290-0045 (JP); KAKUTA, Yoshihisa, Tokyo 105-8449 (JP)
(74) Representative: Cabinet Plasseraud
(86) International application number: PCT/JP2010/068204
(87) International publication number: WO 2011/049027

(57) **Abstract**

The present invention relates to a binuclear ruthenium complex dye having a substituted bipyridyl group and represented by the general formula (1): 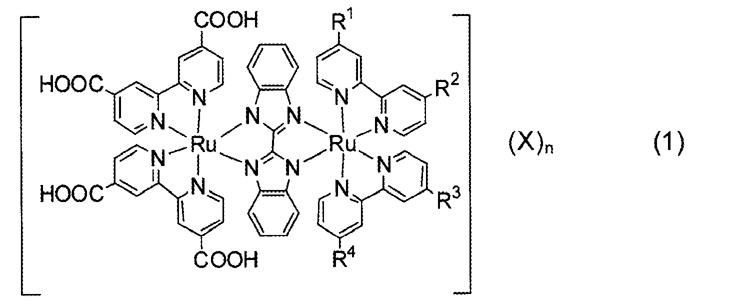 wherein
R¹, R², R³ and R⁴ each independently represents a linear or branched alkyl group having 1 to 30 carbon atoms, with the proviso that at least one of R¹ and R² represents a linear or branched alkyl group having 10 to 30 carbon atoms and at least one of R³ and R⁴ represents a linear or branched alkyl group having 10 to 30 carbon atoms; X represents a counter ion;
n represents a number of the counter ions needed to neutralize a charge of the complex; and
proton(s) (H⁺) of one or more carboxyl groups (-COOH) may dissociate.

## Description

### Technical Field

The present invention relates to a photoelectric conversion element comprising a binuclear ruthenium complex dye having a substituted bipyridyl group, and a photochemical cell comprising the photoelectric conversion element.

### Background Art

A solar battery is greatly expected to serve as a clean renewable energy source, and researches have been conducted for practical application of monocrystalline-silicon, polycrystalline-silicon or amorphous-silicon-based solar batteries and solar batteries comprising, for example, cadmium telluride or indium copper selenide. For the spread of solar battery as a household power source, however, any of these batteries faces many problems to be overcome, including a higher production cost, difficulty in ensuring raw materials, difficulty in recycling, and difficulty in realizing a larger area. Accordingly, there have been proposed solar batteries comprising an organic material in an attempt to achieve a larger area and a lower cost. However, any of these batteries has a conversion efficiency of about 1 %, which falls very short of practical use.

Under such circumstances, Graetzel et al. disclosed a photoelectric conversion element and a solar battery comprising semiconductor particles sensitized by a dye, as well as materials and production technique needed to produce this solar battery in 1991 (see, for example, Non-patent document 1 and Patent document 1). This battery is a wet solar battery comprising a porous titania thin film sensitized by a ruthenium dye as a working electrode. This solar battery has the advantages that the photoelectric conversion element can be provided at a low cost because inexpensive materials can be used without highly purification, and that the solar battery can convert solar light into electricity over a wide visible light wavelength range because the dye used in the solar battery has broad absorption band. However, the conversion efficiency must be further improved for practical use. Thus, there is a need for a dye which has a higher absorption coefficient and absorb light of longer wavelength.

Patent document 2 discloses a mononuclear metal complex containing a dipyridyl ligand, which is a metal complex dye useful for a photoelectric conversion element. In addition, Non-patent document 2 discloses a polynuclear β-diketonate complex dye.

Meanwhile, Patent document 3 discloses a polynuclear complex containing a plurality of metals and a plurality of ligands wherein a bridging ligand (BL) coordinating to the plurality of metals has both a coordination structure with a conjugated heterocyclic ring and a coordination structure without a conjugated heterocyclic ring, which is regarded as a novel polynuclear complex having the excellent photoelectric conversion function of emitting electrons while receiving energy from active ray such as light.

In addition, Patent document 4 discloses a binuclear metal complex having a coordination structure with a conjugated heterocyclic ring, which is a metal complex dye for realizing a photoelectric conversion element having higher photoelectric conversion efficiency.

### Citation List

### Patent Document

Patent document 1: JP-A-1989-220380
Patent document 2: JP-A-2003-261536
Patent document 3: JP-A-2004-359677
Patent document 4: WO 2006/038587 A1
Non-Patent Document

Non-patent document 1: Nature, Vol.353, p.737, 1991
Non-patent document 2: "Current Technology in Dye-sensitized Solar Battery" (CMC Co., LTD., published on May 25, 2001, p.117)

### Summary of Invention

### Problems to be Solved by the Invention

An objective of the present invention is to provide a photoelectric conversion element and a photochemical cell which may exhibit high photoelectric conversion efficiency, and a metal complex dye therefor.

### Means for Solving the Problems

The present invention relates to the following items.

1. A binuclear ruthenium complex dye having a substituted bipyridyl group and represented by the general formula (1):

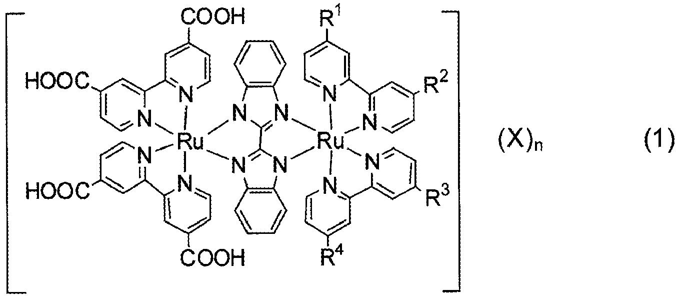

wherein
R¹, R², R³ and R⁴ each independently represents a linear or branched alkyl group having 1 to 30 carbon atoms, with the proviso that at least one of R¹ and R² represents a linear or branched alkyl group having 10 to 30 carbon atoms and at least one of R³ and R⁴ represents a linear or branched alkyl group having 10 to 30 carbon atoms;
X represents a counter ion;
n represents a number of the counter ions needed to neutralize a charge of the complex; and
proton(s) (H⁺) of one or more carboxyl groups (-COOH) may dissociate.

2. A photoelectric conversion element comprising a binuclear ruthenium complex dye represented by the above general formula (1); and a semiconductor particle.

3. A photochemical cell comprising a photoelectric conversion element as described in the above item 2.

4. A photochemical cell comprising a photoelectric conversion element as described in the above item 2 as an electrode, a counter electrode, and an electrolyte layer between them.

5. Aprocess for producing a photoelectric conversion element, comprising a step of:
   immersing a semiconductor particle in a solution containing a binuclear ruthenium complex dye represented by the above general formula (1).

6. Aprocess for producing a photoelectric conversion element, comprising steps of:
   forming a semiconductor layer comprising a semiconductor particle on a conductive support; and
   immersing the semiconductor layer in a solution containing a binuclear ruthenium complex dye represented by the above general formula (1).

### Effect of the Invention

According to the present invention, there may be provided a photoelectric conversion element and a photochemical cell which may exhibit high photoelectric conversion efficiency.

### Description of Embodiments

The binuclear ruthenium complex dye of the present invention, which has a substituted bipyridyl group, is represented by the general formula (1) as described above. In the general formula (1), R¹, R², R³ and R⁴ each independently represents a linear or branched alkyl group having 1 to 30 carbon atoms, with the proviso that at least one of R¹ and R², and at least one of R³ and R⁴ represent a linear or branched alkyl group having 10 to 30 carbon atoms. R¹, R², R³ and R⁴ may be, for example, decyl group, undecyl group, dodecyl group, tridecyl group, tetradecyl group, pentadecyl group, hexadecyl group, heptadecyl group, octadecyl group, nonadecyl group, or eicosyl group, preferably an alkyl group having 12 to 18 carbon atoms, more preferably an alkyl group having 12 to 15 carbon atoms, particularly preferably n-dodecyl group. These groups include various isomers.

A binuclear ruthenium complex dye in which R¹, R², R³ and R⁴ are the same may be preferred in view of ease of synthesis. R¹, R², R³ and R⁴ may be, however, different from each other. In this case, one of R¹ and R², and one of R³ and R⁴ may be a linear or branched alkyl group having 10 to 30 carbon atoms, more preferably having 12 to 18 carbon atoms; and the others may be a linear or branched alkyl group having 1 to 9 carbon atoms, for example, methyl group, ethyl group, propyl group, butyl group, pentyl group, hexyl group, heptyl group, octyl group, or nonyl group. These groups include various isomers.

X represents a counter ion. X may be, for example, hexafluorophosphate ion, perchlorate ion, tetraphenylborate ion, tetrafluoroborate ion, trifluoromethanesulfonate ion, thiocyanate ion, sulfate ion, nitrate ion, or halide ion. X may be preferably hexafluorophosphate ion, tetrafluoroborate ion, trifluoromethanesulfonate ion, nitrate ion or halide ion, and more preferably hexafluorophosphate ion, tetrafluoroborate ion, nitrate ion or iodide ion. In addition, n represents a number of the counter ions needed to neutralize a charge of the complex.

The binuclear ruthenium complex dye of the present invention, which has a substituted bipyridyl group, may be prepared by reacting two different mononuclear ruthenium complexes as shown in the following scheme, for example, by reference to WO2006/038587.

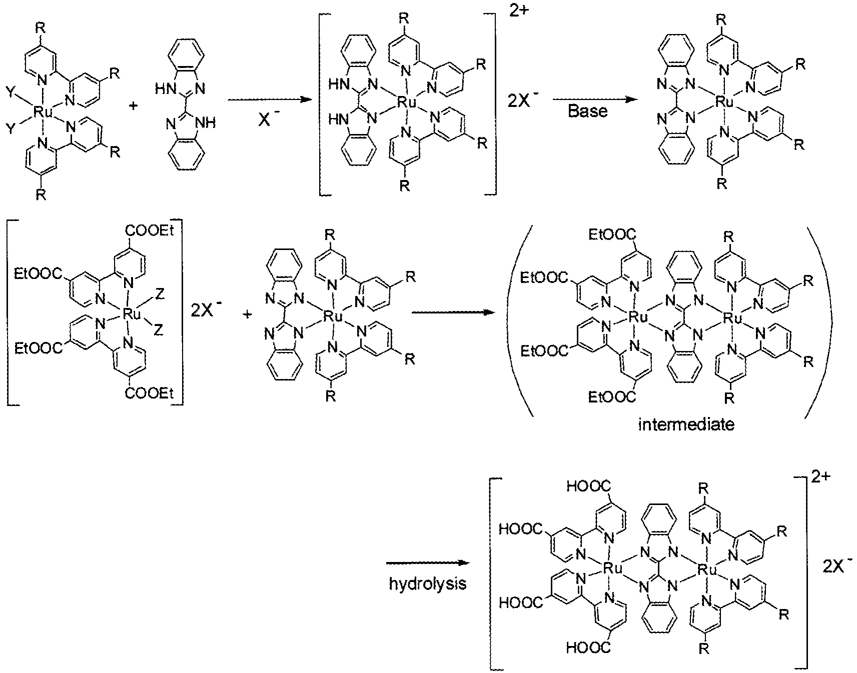

wherein R represents a linear or branched alkyl group having 10 to 30 carbon atoms, X represents a monovalent anion being a counter ion, and Y represents a halogen atom. Z represents a neutral ligand such as water, acetone, methanol, ethanol, tetrahydrofuran, diethyl ether and acetonitrile.

The counter ion (X) is not limited to a monovalent anion, and other complex dyes may be synthesized in the same way as described above. A binuclear ruthenium complex dye in which R¹, R², R³ and R⁴ are not the same may be also synthesized in the same way as described above.

One of the mononuclear ruthenium complexes may be synthesized via a mononuclear ruthenium complex precursor. The synthetic intermediate, i.e. the mononuclear ruthenium complex precursor represented by the general formula (2):

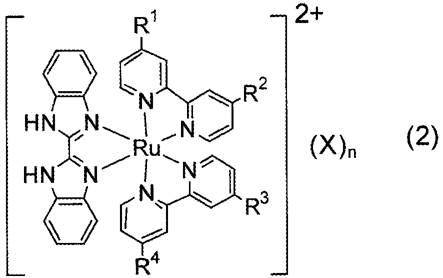

wherein R¹, R², R³, R⁴, X and n are defined as above, and the mononuclear ruthenium complex represented by the general formula (3):

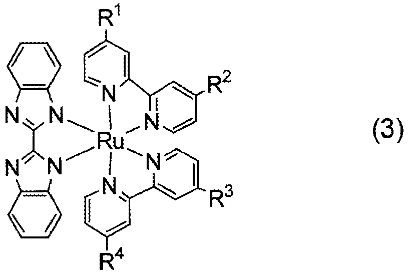

wherein R¹, R², R³ and R⁴ are defined as above,
are novel compounds.

The mononuclear ruthenium complex represented by the general formula (3) may have a NH-proton, as represented by the general formula (4):

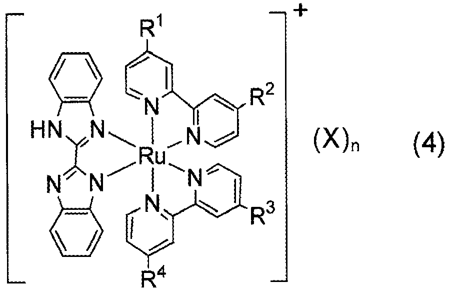

wherein R¹, R², R³, R⁴, X and n is defined as above.

In the binuclear ruthenium complex dye of the present invention, which has a substituted bipyridyl group, proton(s) (H⁺) of one or more carboxyl groups (-COOH) may dissociate. The dissociation of proton (H⁺) may be achieved mainly by adjusting the pH of the binuclear ruthenium complex dye solution.

The photoelectric conversion element of the present invention comprises the binuclear ruthenium complex dye as described above, and a semiconductor particle. The binuclear ruthenium complex dye is adsorbed on the surface of the semiconductor particle, and the semiconductor particle is sensitized with the ruthenium complex dye.

More specifically, the photoelectric conversion element of the present invention comprises a semiconductor particle sensitized with the ruthenium complex dye, which is fixed on a conductive support (electrode).

A conductive electrode may be preferably a transparent electrode, which is formed on a transparent substrate. Examples of a conducting agent include metals such as gold, silver, copper, platinum and palladium; indium oxide-based compounds, typified by tin-doped indium oxide (ITO); tin oxide-based compounds, typified by fluorine-doped tin oxide (FTO); and zinc oxide-based compounds.

Examples of the semiconductor particle include titanium oxide, zinc oxide and tin oxide. The other examples may include indium oxide; niobium oxide; tungsten oxide; vanadium oxide; composite oxide semiconductors such as strontium titanate, calcium titanate, barium titanate and potassium niobate; cadmium or bismuth sulfide; cadmium selenide or telluride; and gallium phosphide or arsenide. The semiconductor particle may be preferably an oxide, particularly preferably, for example, titanium oxide, zinc oxide, tin oxide, or a mixture comprising at least one of these oxides.

Aprimary particle size of the semiconductor particle is not limited, but it is generally from 1 nm to 5,000 nm, preferably from 2 nm to 500 nm, particularly preferably from 5 nm to 400 nm.

The binuclear ruthenium complex dye may be adsorbed onto the semiconductor particle, for example, by forming a semiconductor layer which comprises a semiconductor particle (semiconductor particle film) on a conductive support; and then immersing the semiconductor layer in a solution containing the binuclear ruthenium complex dye. The semiconductor layer may be formed by applying a paste of semiconductor particle onto a conductive support; and then calcining the paste. Subsequently, the conductive support on which the semiconductor layer is formed is immersed in a solution containing the dye; and then washed and dried.

Examples of a solvent for the dye solution include water; alcohols such as methanol, ethanol, isopropyl alcohol, t-butyl alcohol and ethylene glycol; nitriles such as acetonitrile and propionitrile; amides such as N,N-dimethylacetamide and N,N-dimethylformamide; ureas such as N-methylpyrrolidone; and sulfoxides such as dimethylsulfoxide. The solvent to be used may be preferably water, an alcohol or a nitrile, more preferably water, ethanol, isopropyl alcohol, t-butanol or acetonitrile. These solvents may be used alone or in combination of two or more.

The concentration of the dye in the solution may be preferably from 0.001 mmol/l to the saturation concentration of the complex dye of the present invention, more preferably from 0.001 mmol/l to 100 mmol/l, particularly preferably from 0.01 mmol/l to 10 mmol/l, and more preferably from 0.05 mmol/l to 1.0 mmol/l.

The dye solution may contain a compound having a steroid skeleton such as cholic acid, deoxycholic acid and chenodeoxycholic acid.

The temperature at which the dye is adsorbed onto a semiconductor particle is generally from 0 °C to 80 °C, preferably from 20 °C to 40 °C. The time period for which the dye is adsorbed onto a semiconductor particle (time period for which a semiconductor particle is immersed in the dye solution) may be appropriately selected depending on the type of the binuclear ruthenium complex dye, the concentration of the dye in the solution, and other conditions.

The photochemical cell of the present invention comprises the photoelectric conversion element of the present invention as described above. More specifically, it comprises the photoelectric conversion element of the present invention as described above, and a counter electrode as electrodes; and comprises an electrolyte layer between the electrodes. At least one of the electrode, which is the photoelectric conversion element of the present invention, and the counter electrode is a transparent electrode.

The counter electrode functions as a cathode when it is combined with the photoelectric conversion element to form a photochemical cell. Although a substrate on which a conductive layer is formed may be used as a counter electrode, like the conductive electrode as described above, a substrate is not necessarily required for a counter electrode, for example, a metal plate itself may be used as a counter electrode. Examples of a conducting agent to be used for the counter electrode include metals such as platinum and carbon; and conductive metal oxides such as fluorine-doped tin oxide.

The electrolyte (redox couple) may be selected from any known materials without limitations. Examples of the electrolyte to be used include a combination of iodine and an iodide (for example, metal iodides such as lithium iodide and potassium iodide, or iodides of a quaternary ammonium compound such as tetrabutylammonium iodide, tetrapropylammonium iodide, pyridinium iodide and imidazolium iodide); a combination of bromine and a bromide; a combination of chlorine and a chloride; a combination of an alkylviologen and a reductant thereof; quinone/hydroquinone; transition metal ion pair such as iron (II) ion/iron (III) ion, copper (I) ion/copper (II) ion, manganese(II) ion/manganese(III) ion, and cobalt (II) ion/cobalt (III) ion; a combination of complex ions such as ferrocyanide/ferricyanide, cobalt (II) tetrachloride/cobalt (III) tetrachloride, cobalt (II) tetrabromide/cobalt (III) tetrabromide, iridium(II) hexachloride/iridium (III) hexachloride, ruthenium (II) hexacyanide/ruthenium (III) hexacyanide, rhodium(II) hexachloride/rhodium(III) hexachloride, rhenium (III) hexachloride/rhenium (IV) hexachloride, rhenium (IV) hexachloride/rhenium (V) hexachloride, osmium (III) hexachloride/osmium (IV) hexachloride, and osmium (IV) hexachloride/osmium (V) hexachloride; complexes formed with a transition metals such as cobalt, iron, ruthenium, manganese, nickel and rhenium, and a conjugated heterocyclic ring and derivative thereof such as bipyridine and derivative thereof, terpyridine and derivative thereof, and phenanthroline and derivative thereof; complexes of cyclopentadiene or derivative thereof and a metal such as ferrocene/ferrocenium ion, cobaltocene/cobaltocenium ion, and ruthenocene/ruthenocenium ion; and porphyrin compounds. A preferable electrolyte may be a combination of iodine and lithium iodide or an iodide of a quaternary ammonium compound. The electrolyte may be a liquid in which the electrolyte is dissolved in an organic solvent, a molten salt, a so-called gel electrolyte in which the electrolyte is impregnated in a polymer matrix, or a solid electrolyte.

Examples of a solvent for the electrolyte solution to be used include, but not limited to, water, alcohols, nitriles, chain ethers, cyclic ethers, chain esters, cyclic esters, chain amides, cyclic amides, chain sulfones, cyclic sulfones, chain ureas, cyclic ureas, and amines. These solvents may be used alone or in combination of two or more.

The photochemical cell of the present invention may be produced by any conventional process.

For example, the photochemical cell of the present invention may be produced as follows. As described above, a paste of semiconductor particle such as an oxide is applied onto a transparent electrode, and then calcined the paste, to form a semiconductor particle film. In the case where the semiconductor particle film is titania, the paste is calcined at a temperature of from 450 °C to 500 °C for 30 minutes, for example. The transparent electrode with the semiconductor particle film is immersed in a dye solution (a solution containing the binuclear ruthenium complex dye of the present invention) to fix the dye on the semiconductor particles, thereby producing a photoelectric conversion element. Then, the photoelectric conversion element is combined with a transparent electrode on which platinum or carbon is vapor-deposited as a counter electrode, and an electrolyte is placed between them, to produce the photochemical cell of the present invention.

### EXAMPLES

The present invention will be described in more detail below with reference to the Examples. However, the scope of the present invention is not limited to the Examples. Abbreviations used in the Examples are as follows:

Etcbpy; 2,2'-bipyridine-4,4'-dicarboxylic acid diethyl ester,
H₂dcbpy; 2,2'-bipyridine-4,4'-dicarboxylic acid,
OTf; trifluoromethanesulfonate ion,
bpy; 2,2'-bipyridine,
ddbpy; 4,4'-di-dodecyl-2,2'-bipyridine,
cod; 1,5-cyclooctadiene,
BiBzImH₂; 2,2'-bibenzimidazole,
dtbpy; 4,4'-di-t-butyl-2,2'-bipyridine,
dnbpy; 4,4'-di-nonyl-2,2'-bipyridine,
dmbpy; 4,4'-di-methyl-2,2'-bipyridine,
mdbpy; 4,4'-dodecylmethyl-2,2'-bipyridine.

### Example 1A-1

### (Synthesis of the mononuclear ruthenium complex (M-1) [Ru(Etcbpy)₂ (H₂O)₂ ](OTf)2)

Under nitrogen atmosphere, into a 500 mL three-necked flask were placed commercially available H₂dcbpy (5.44 g, 22.3 mmol), concentrated sulfuric acid (10 mL) and ethanol (130 mL). The mixture was refluxed overnight. After cooling down, the mixture was neutralized. The precipitate was collected by filtration, and washed with hot water. And then, the precipitate was recrystallized with ethanol/water (95:5) and collected by filtration, to provide Etcbpy (4.92 g).

Subsequently, under argon atmosphere, into a 1000 mL three-necked flask were placed commercially available ruthenium chloride (1.18 g, 4.51 mmol), Etcbpy (2.64 g, 8.79 mmol) and ethanol (500 mL). The mixture was refluxed for 7 hours. After cooling down, the precipitate was collected by filtration, to provide [Ru(Etcbpy)₂Cl₂] (1.64 g). In addition, the resultant filtrate was concentrated under reduced pressure. And then, a 2 mol/L hydrochloric acid (300 mL) was added to the resultant concentrate, and the mixture was stirred at room temperature for 5 minutes. After completion of stirring, the insoluble substance was collected by filtration, and washed with water. And then, the insoluble substance was recrystallized with ethanol/dichloromethane (10:3), to provide [Ru(Etcbpy)₂Cl₂] (1.34 g). Consequently, 2.98 g of [Ru(Etcbpy)₂Cl₂] was obtained in total.

Subsequently, into a 200 mL three-necked flask were placed [Ru(Etcbpy)₂Cl₂] (1.37 g, 1.77 mmol), silver trifluoromethanesulfonate (1.09 g, 4.25 mmol) and dichloromethane (140 mL). The mixture was stirred at room temperature for 1 hour. After allowing the mixture to stand overnight, the precipitate was collected by filtration. The resultant filtrate was concentrated, and then stirred in diethyl ether at room temperature for 5 minutes. After completion of stirring, the insoluble substance was collected by filtration, to provide the mononuclear ruthenium complex (M-1) [Ru(Etcbpy)₂ (H₂O)₂ ](OTf)₂ (1.62 g).

### Example 1A-2

### (Synthesis of the mononuclear ruthenium complex (M-2) [(BiBzIm)Ru(ddbpy)₂] {the complex of the formula (3) in which R¹, R², R³, R⁴ = n-dodecyl group})

Under argon atmosphere, into a 100 mL three-necked flask were placed ddbpy (0.8930 g, 1.812 mmol), [Ru(cod)Cl₂]ₙ (0.250 g, 0.888 mmol) and N,N-dimethylformamide (93 mL). After deaeration, the mixture was refluxed for 44 minutes while being stirred under irradiation with 2.45 GHz microwave. After cooling down, the mixture was concentrated under reduced pressure. The resultant concentrate was suspended in water (40 mL), and then the precipitate was collected by suction filtration, and washed with water, to provide [Ru(ddbpy)₂Cl₂] (0.857 g).

Subsequently, under argon atmosphere, into a 20 mL three-necked flask were placed Ru(ddbpy)₂Cl₂ (0.857 g, 0.740 mmol), BiBzImH₂ (0.191 g, 0.814 mmol) and ethylene glycol (30 mL). The mixture was refluxed for 26 minutes while being stirred under irradiation with 2.45 GHz microwave. After cooling down, to the mixture were added ethanol (65 mL), water (20 mL) and ammonium hexafluorophosphate (0.482 g, 2.957 mmol) dissolved in water (8 mL). The resultant mixture was stirred at room temperature for 1 hour. And then, the precipitate was collected by filtration, and washed with water, to provide [(BiBzImH₂)Ru(ddbpy)₂](PF₆)₂ (0.988 g).

Subsequently, under argon atmosphere, into a 20 mL Schlenk flask were placed [(BiBzImH₂)Ru(ddbpy)₂](PF₆)₂ (0.959 g, 0.595 mmol), methanol (6.8 mL) and a 10 % lithium methoxide methanol solution (2.26 mL, 5.951 mmol). After deaeration, the mixture was refluxed for 1 hour. After cooling down, the residue was collected by suction filtration, and washed with a 0.64 mol/L lithium methoxide methanol solution (which had the same concentration as the reaction solution), to provide the mononuclear ruthenium complex (M-2) [(BiBzIm)Ru(ddbpy)₂] (0.213 g).

### Example 1

### (Synthesis of the binuclear ruthenium complex dye (1a: the product isolated at pH 2.8, or pH 2.8 isolate) [(H₂dcbpy)(Hdcbpy)Ru(BiBzIm)Ru(ddbpy)₂](PF₆) {the complex of the formula (1) in which R¹, R², R³, R⁴ = n-dodecyl group})

Under argon atmosphere, into a 100 mL three-necked flask were placed the mononuclear ruthenium complex (M-2) (0.400 g, 0.304 mmol), the mononuclear ruthenium complex (M-1) (0.309 g, 0.304 mmol) and N,N-dimethylformamide (50 mL). After deaeration, the mixture was refluxed for 44 minutes while being stirred under irradiation with 2.45 GHz microwave. After cooling down, the mixture was concentrated under reduced pressure. And then, a 0.2 mol/L sodium hydroxide aqueous solution (70 mL) was added to the resultant concentrate, and the mixture was heated at 100 °C for 1 hour. After cooling down, the insoluble substance was removed from the mixture by suction filtration, and washed with water. And then, a 1.7 mol/L hexafluorophosphoric acid aqueous solution was added to the resultant filtrate until pH became 1.8. After allowing the mixture to stand overnight, the precipitate was collected by suction filtration, washed with a hexafluorophosphoric acid aqueous solution at pH 1.8, and purified by preparative chromatography. The purified product was concentrated under reduced pressure. And then, water (50 mL) and a 1 mol/L sodium hydroxide aqueous solution (0.8 mL) was added to the resultant concentrate. And then, a 0.5 mol/L hexafluorophosphoric acid aqueous solution was added to the resultant mixture until pH became 2.8, and the mixture was left in the refrigerator overnight. The precipitate was collected by suction filtration, washed with a hexafluorophosphoric acid aqueous solution at pH 2.8, and then dried under vacuum, to provide the binuclear ruthenium complex dye (1a: pH 2.8 isolate) (0.087 g).

### Example 2

### (Synthesis of the binuclear ruthenium complex dye (2a: the product isolated at pH 3.8, or pH 3.8 isolate) [(H₂dcbpy)(Hdcbpy)Ru(BiBzIm)Ru(ddbpy)₂](PF₆) {the complex of the formula (1) in which R¹, R², R³, R⁴ = n-dodecyl group})

The binuclear ruthenium complex dye (2a: pH 3.8 isolate) (0.063 g) was synthesized in the same way as in Example 1, except that the pH of the mixture adjusted with a 0.5 mol/L hexafluorophosphoric acid aqueous solution and the pH of the hexafluorophosphoric acid aqueous solution used as the wash liquid were changed from 2.8 to 3.8.

### Example 3-1

### (Production of a porous titania electrode)

A titania paste PST-18NR for a transparent layer and a titania paste PST-400C for a diffusion layer, both of which were made by Catalysts & Chemicals Industries Co., Ltd., were applied onto a transparent conductive glass electrode, which was made by Asahi Glass Co., Ltd., using a screen printer. The film thus obtained was aged in an atmosphere at 25 °C and 60 %RH for 5 minutes, and then the aged film was calcined at 450 °C for 30 minutes. After the film was cooled down, the same procedure was repeated to achieve the predetermined thickness, thereby producing a 16 mm² porous titania electrode.

### Example 3-2

### (Production of a dye-adsorbed porous titania electrode)

The porous titania electrode was immersed in a 0.3 mmol/L binuclear ruthenium complex dye solution [solvent: mixed solvent of t-butanol and acetonitrile (1:1)] at 30 °C for the predetermined time period, and then the electrode was dried, to provide a dye-adsorbed porous titania electrode.

### Example 3-3

### (Production of a photochemical cell)

The dye-adsorbed porous titania electrode thus obtained was placed on a platinum plate (counter electrode). Subsequently, an electrolyte (a solution prepared by dissolving lithium iodide, iodine, 4-t-butylpyridine and 1,2-dimethyl-3-propylimidazolium iodide in 3-methoxypropionitrile in an amount of 0.1 mol/L, 0.05 mol/L, 0.5 mol/L and 0.6 mol/L, respectively) was poured into a gap between these electrodes by the capillary action, to provide a photochemical cell.

### Example 3-4

### (Measurement of conversion efficiency)

The photoelectric conversion efficiency of the photochemical cell thus obtained was measured under irradiation with artificial solar light at 100 mW/cm², using a solar simulator made by EKO Instruments Co., Ltd.

Table 1 shows the results of the measurements of the conversion efficiencies of the binuclear ruthenium complex dyes (1a) and (2a) of the present invention, and, as the comparative dyes, the comparative dye (1) [(H₂dcbpy)(Hdcbpy)Ru(BiBzIm)Ru(bpy)₂](PF₆) {the complex of the formula (1) in which R¹, R², R³, R⁴ = H}, the comparative dye (2) [(H₂dcbpy)(Hdcbpy)Ru(BiBzIm)Ru(dtbpy)₂](PF₆) {the complex of the formula (1) in which R¹, R², R³, R⁴ = t-butyl group}, the comparative dye (3) [(H₂dcbpy)(Hdcbpy)Ru(BiBzIm)Ru(dnbpy)₂](PF₆) {the complex of the formula (1) in which R¹, R², R³, R⁴ = n-nonyl group}, which were binuclear ruthenium complex dyes, and the comparative dye (4) N719; Ruthenium 535-bisTBA, made by Solaronics, Inc., which was a mononuclear ruthenium complex dye. The comparative dyes (1) - (3) were synthesized by reference to W02006/038587.

**Table 1**

| Complex dye | Number of carbon atoms in substituent | Conversion efficiency (%) |
|---|---|---|
| Comparative dye (1) | 0 | 6.3 |
| Comparative dye (2) | 4 | 7.4 |
| Comparative dye (3) | 9 | 7.1 |
| Dye (1a) (Example 1) | 12 | 7.9 |
| Dye (2a) (Example 2) | 12 | 8.2 |
| Comparative dye (4) N-719 | -- | 7.8 |

As can be seen from the results given in Table 1, the binuclear ruthenium complex dyes (1a) and (2a) of the present invention, which had alkyl groups having 12 carbon atoms, exhibited higher conversion efficiency than the binuclear ruthenium complex dye (comparative dye (1)) which had no substituents (alkyl groups) and the binuclear ruthenium complex dyes (comparative dyes (2), (3)) which had alkyl groups having 4 carbon atoms or 9 carbon atoms, and the existing high-efficiency mononuclear ruthenium complex dye (comparative dye (4); N719). In addition, the binuclear ruthenium complex dyes (1a) and (2a) of the present invention had greater durability than the existing high-efficiency mononuclear ruthenium complex dye (comparative dye (4); N719). The results indicate that the binuclear ruthenium complex dyes (1a) and (2a) of the present invention may be promising for producing a high-performance photochemical cell.

### Example 4A-1

### (Synthesis of the mononuclear ruthenium complex (M-1') [Ru(H₂dcbpy)₂Cl₂])

Into a 500 mL three-necked flask were placed ruthenium chloride tri-hydrate (which was adjusted to be trivalent) (3.229 g, 12.35 mmol), H₂dcbpy (5.731 g, 23.47 mmol) and N,N-dimethylformamide (300 mL). After deaeration, the mixture was refluxed for 45 minutes while being stirred under irradiation with 2.45 GHz microwave. After cooling down, the reaction liquid was subjected to suction filtration, and the resultant filtrate was concentrated under reduced pressure. The resultant concentrate was suspended in the mixed solvent of acetone and diethyl ether (acetone: diethyl ether = 1:4), and then the precipitate was collected by suction filtration. The resultant precipitate was suspended in a 2 mol/L hydrochloric acid (300 mL), and then the resultant suspension was stirred with ultrasonic for 30 minutes, and further stirred at room temperature for 30 minutes. And then, the precipitate was collected by suction filtration, to provide the mononuclear ruthenium complex (M-1') [Ru(H₂dcbpy)₂Cl₂] (7.747 g).

### Example 4A-2

### (Synthesis of the mononuclear ruthenium complex (M-2') [(BiBzIm)Ru(mdbpy)₂] {the complex of the formula (3) in which R¹, R³ = methyl group, R², R⁴ = n-dodecyl group})

Under nitrogen atmosphere, into a 200 mL three-necked flask were placed dmbpy (0.965 g, 5.232 mmol) and tetrahydrofuran (75 mL). While the flask, which contained the solution, was cooled with ice, a 1.14 M solution of lithium di-isopropylamide in hexane/tetrahydrofuran (4.4 mL, 5.016 mmol) was added dropwise to the solution little by little, and the resultant solution was stirred for 1 hour. And then, while the flask was cooled with ethanol/dry ice, 1-bromoundecane (1.293 g, 5.499 mmol) was added dropwise to the solution little by little, and the resultant solution was stirred for 2 hours. Subsequently, the solution was stirred at room temperature overnight. After completion of stirring, methanol (5 mL) was added to the reaction solution, while the flask, which contained the reaction solution, was cooled with ice. The reaction solution was subjected to solvent fractionation with diethyl ether/water, and the diethyl ether phase was dried with sodium sulfate. And then, the diethyl ether phase was concentrated under reduced pressure, and the resultant concentrate was purified by column chromatography with basic alumina (hexane: ethyl acetate = 100:1 → 50:1), to provide mdbpy (0.486 g).

Subsequently, under nitrogen atmosphere, into a 200 mL three-necked flask were placed mdbpy (0.464 g, 1.372 mmol), [Ru(cod)Cl₂]ₙ (0.188 g, 0.671 mmol) and N,N-dimethylformamide (70 mL). After deaeration, the mixture was refluxed for 24 minutes while being stirred under irradiation with 2.45 GHz microwave. After cooling down, the mixture was concentrated under reduced pressure. The resultant concentrate was suspended in ethanol (20 mL) and water (10 mL), and then the precipitate was collected by suction filtration, and washed with the mixed solvent of ethanol and water (ethanol: water = 2:1), to provide [Ru(mdbpy)₂Cl₂] (0.292 g).

Subsequently, under nitrogen atmosphere, into a 20 mL Schlenk flask were placed Ru(mdbpy)₂Cl₂ (0.146 g, 0.172 mmol), BiBzImH₂ (0.045 g, 0.192 mmol) and ethylene glycol (7 mL). The mixture was refluxed for 14 minutes while being stirred under irradiation with 2.45 GHz microwave. After cooling down, ethanol (7 mL) was added to the mixture, and then the filtrate was collected by suction filtration. And then, to the filtrate were added water (11 mL) and ammonium hexafluorophosphate (0.112 g, 0.687 mmol) dissolved in water (2 mL). The resultant mixture was stirred at room temperature for 1 hour. And then, the precipitate was collected by filtration, and washed with water, to provide [(BiBzImH₂)Ru(mdbpy)₂](PF₆)₂ (0.200 g).

Subsequently, under nitrogen atmosphere, into a 20 mL Schlenk flask were placed [(BiBzImH₂)Ru(mdbpy)₂](PF₆)₂ (0.180 g, 0.139 mmol), methanol (6 mL) and a 10 % lithium methoxide methanol solution (1.06 mL, 2.791 mmol). After deaeration, the mixture was refluxed for 1 hour. After cooling down, the filtrate was collected by suction filtration, to provide the mixture (0.304 g) of the mononuclear ruthenium complex (M-2') [(BiBzIm)Ru(mdbpy)₂] and lithium methoxide.

### Example 4-1

### (Synthesis of the binuclear ruthenium complex dye (3a) {the complex of the formula (1) in which R¹, R³ = methyl group, R², R⁴ = n-dodecyl group})

Into a 50 mL sample tube were placed the mononuclear ruthenium complex (M-2') (0.304 g, 0.139 mmol; as the mixture of the complex and lithium methoxide), water (30 mL) and the mononuclear ruthenium complex (M-1') (0.101 g, 0.145 mmol). The mixture was stirred for 30 minutes. And then, N,N-dimethylformamide (30 mL) was added to the reaction liquid. The mixture was transferred into a 200 mL three-necked flask. Into the flask was added N,N-dimethylformamide (60 mL). After deaeration, the mixture was refluxed for 34 minutes while being stirred under irradiation with 2.45 GHz microwave. The reaction liquid was concentrated under reduced pressure. The resultant concentrate was suspended in water (50 mL) added thereto, and then the precipitate was collected by suction filtration, to provide the binuclear ruthenium complex dye (3a). ESI-MS(-): m/z: 798.25.

### Example 4-2

### (Synthesis of the binuclear ruthenium complex dye (3a) {the complex of the formula (1) in which R¹, R³ = methyl group, R², R⁴ = n-dodecyl group})

Into a 30 mL Schlenk flask were placed [(BiBzImH₂)Ru(mdbpy)₂](PF₆)₂ (0.143 g, 0.101 mmol), N,N-dimethylformamide (5 mL), tert-butoxy potassium (0.023 g, 0.209 mmol) and the mononuclear ruthenium complex (M-1) (0.113 g, 0.111 mmol). After deaeration, the mixture was refluxed for 24 minutes while being stirred under irradiation with 2.45 GHz microwave. After cooling down, the reaction liquid was concentrated under reduced pressure. And then, a 0.2 mol/L sodium hydroxide aqueous solution (24 mL) was added to the resultant concentrate, and the mixture was heated at 100 °C for 2 hours. After cooling down, the insoluble substance was removed from the mixture by suction filtration, and washed with water. And then, a 0.74 mol/L hexafluorophosphoric acid aqueous solution was added to the resultant filtrate until pH became 3.8. After allowing the mixture to stand overnight, the precipitate was collected by suction filtration, washed with a hexafluorophosphoric acid aqueous solution at pH 3.8, and then dried under vacuum. The dried product was washed with the mixture of acetone and diethyl ether (acetone: diethyl ether = 1:8). The precipitate was collected by suction filtration, and then dried under vacuum, to provide the binuclear ruthenium complex dye (3a).

### Industrial Applicability

According to the present invention, there may be provided a photoelectric conversion element and a photochemical cell which may exhibit high photoelectric conversion efficiency.

## Claims

1. A binuclear ruthenium complex dye having a substituted bipyridyl group and represented by the general formula (1): 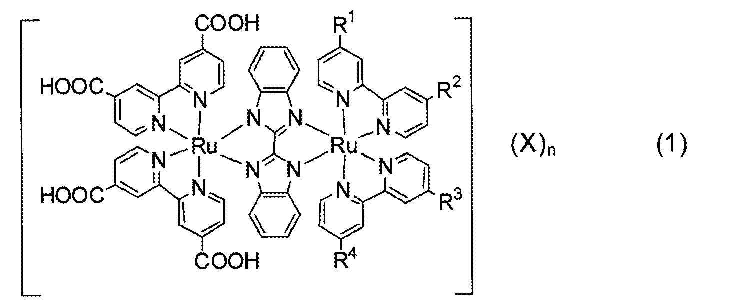 wherein
R¹, R², R³ and R⁴ each independently represents a linear or branched alkyl group having 1 to 30 carbon atoms, with the proviso that at least one of R¹ and R² represents a linear or branched alkyl group having 10 to 30 carbon atoms and at least one of R³ and R⁴ represents a linear or branched alkyl group having 10 to 30 carbon atoms;
X represents a counter ion;
n represents a number of the counter ions needed to neutralize a charge of the complex; and
proton(s) (H⁺) of one or more carboxyl groups (-COOH) may dissociate.

2. A binuclear ruthenium complex dye according to Claim 1, wherein in the general formula (1), R¹, R², R³ and R⁴ each independently represents a linear or branched alkyl group having 10 to 30 carbon atoms.

3. A photoelectric conversion element comprising a binuclear ruthenium complex dye according to Claim 1; and a semiconductor particle.

4. A photoelectric conversion element according to Claim 3, wherein the semiconductor particle is at least one selected from the group consisting of titanium oxide, zinc oxide and tin oxide.

5. A photochemical cell comprising a photoelectric conversion element according to Claim 3.

6. A photochemical cell comprising a photoelectric conversion element according to Claim 3 as an electrode, a counter electrode, and an electrolyte layer between them.

7. Aprocess for producing a photoelectric conversion element, comprising a step of:
immersing a semiconductor particle in a solution containing a binuclear ruthenium complex dye according to Claim 1.

8. Aprocess for producing a photoelectric conversion element, comprising steps of:
forming a semiconductor layer comprising a semiconductor particle on a conductive support; and
immersing the semiconductor layer in a solution containing a binuclear ruthenium complex dye according to Claim 1.

9. A mononuclear ruthenium complex precursor represented by the general formula (2): 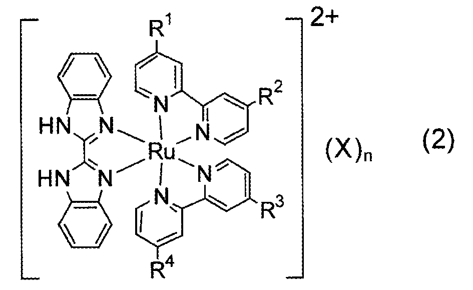 wherein R¹, R², R³, R⁴, X and n are defined as above.

10. A mononuclear ruthenium complex represented by the general formula (3): 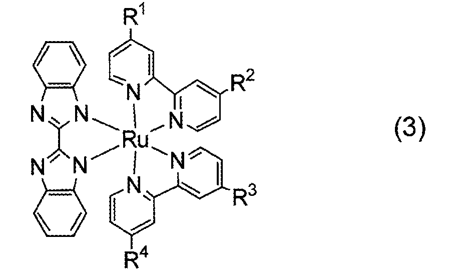 wherein R¹, R², R³ and R⁴ are defined as above.

11. A mononuclear ruthenium complex represented by the general formula (4): 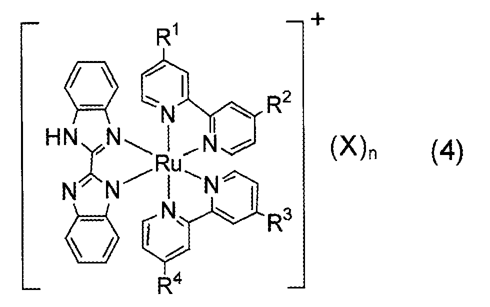 wherein R¹, R², R³, R⁴, X and n is defined as above.
